# EUROPEAN PATENT APPLICATION

(11) **EP 4 520 247 A1**
(43) Date of publication of application: **12.03.2025**
(21) Application number: 24190014.1
(22) Date of filing: 22.07.2024
(51) Int. Cl.: A61B 1/00, A61B 1/267, A61B 13/00, A61M 16/04

(54) **TONGUE DEPRESSOR**

(30) Priority: 07.09.2023 TW 112134081
(71) Applicant: Chen, Tien-Sheng, Taipei City 112 (TW)
(72) Inventor: Chen, Tien-Sheng, Taipei City 112 (TW)
(74) Representative: Viering, Jentschura & Partner mbB Patent- und Rechtsanwälte

(57) **Abstract**

A tongue depressor (1, 1a, 1b) for facilitating the placement of a laryngeal mask (80) is disclosed. The tongue depressor (1, 1a, 1b) includes a flexible main body (10); the flexible main body (10) is long and flat with a width W and a thickness D, wherein 1.5 cm < W < 3.5 cm and 0.05 mm < D < 2 mm. The flexible main body (10) has a bent state (S2) and a straight state (S1). With the above structure, when the flexible main body (10) is put into the oral cavity (90) of the human body in the straight state (S1), an external force (F) is applied to the flexible main body (10) and then the flexible main body (10) transitions from the straight state (S 1) to the bent state(S2) such that the flexible main body (10) depresses the tongue base (94) to form a channel (100) with the upper jaw of the human oral cavity (90), through which the laryngeal mask (80) can enter the human pharynx (93).

## Description

### BACKGROUND OF THE INVENTION

### Field of the Invention

The present disclosure relates to a tongue depressor, especially to a tongue depressor with a flexible main body for assisting a placement of a laryngeal mask.

### Description of the Related Art

It is well known in medical practice that laryngeal masks are inserted by medical personnel into a patient's pharynx to access the tracheal opening for oxygen delivery. However, due to the curved structure of the human palate and pharynx, when medical personnel attempt to insert the laryngeal mask into the pharynx, the front end of the laryngeal mask often is compressed and deformed due to the pushing force when it contacts the patient's oral cavity or pharynx. Additionally, the soft tissue of the tongue may become lodged in the opening of the laryngeal mask, making it difficult for medical personnel to apply force or push the laryngeal mask into place. Hence, there is a need for improvement.

### SUMMARY OF THE INVENTION

The object of the present disclosure is to provide a tongue depressor with a flexible main body for assisting a placement of a laryngeal mask.

In order to achieve the above object, a tongue depressor of the present disclosure for assisting a placement of a laryngeal mask includes a flexible main body in an elongated and flattened shape having a width W and a thickness D, wherein 1.5 cm < W < 3.5 cm and 0.05 mm < D < 2 mm. The flexible main body has a bent state and a straight state, whereby, after the flexible main body is placed in a human oral cavity while the tongue depressor is in the straight state, an external force is applied to the flexible main body and then the flexible main body transitions from the straight state S1 to the bent state S2. Thus, when the flexible main body presses the tongue, a passage between the tongue and the human palate is formed, and the laryngeal mask can move forward along the flexible main body without being deformed by compression. Consequently, the soft tissue of the tongue can be prevented from entering the opening of the laryngeal mask and the laryngeal mask can smoothly enter the human pharynx via the passage.

The tongue depressor of the present disclosure has a flexible main body, and after the flexible main body has been inserted into the human oral cavity, an external force can be applied to the flexible main body to cause the flexible main body to transition from the straight state S1 to the bent state S2. Then a passage is formed among the tongue depressor in the bent state, the hard palate, the soft palate, and the cervical vertebrae, via which the laryngeal mask can enter the human pharynx. Additionally, because the concave arc surface of the tongue depressor is shaped with two slightly higher opposite edges and a slightly lower center, the present disclosure allows the opening of the laryngeal mask to contact the concave arc surface and to enter the human pharynx by sliding along the concave arc surface via the passage. The shape characteristics of the concave arc surface control the direction of movement of the laryngeal mask into the human throat and prevent deformation and obstruction of the laryngeal mask due to compression, while simultaneously the tongue soft tissue is prevented from entering the opening of the laryngeal mask. Thereby, the difficulty of laryngeal mask placement is reduced and the success rate of placement is increased. This addresses the issues of the prior art, where the curved structure of the human upper palate and pharynx often cause the front end of the laryngeal mask to deform due to compression when medical personnel attempt to insert the laryngeal mask into the pharynx. In the prior art, when the deformed laryngeal mask comes into contact with the inside of the patient's oral cavity or pharynx such that the tongue soft tissue becomes lodged in the opening of the laryngeal mask, medical are personnel unable to apply force on or push the laryngeal mask to move it forward.

### BRIEF DESCRIPTION OF THE DRAWINGS

FIG. 1 illustrates a schematic view of the first embodiment of the tongue depressor of the present disclosure in the straight state.
FIG. 2 illustrates a schematic view of the second embodiment of the tongue depressor of the present disclosure in the straight state.
FIG. 3 illustrates a schematic view of the second embodiment of the tongue depressor of the present disclosure in the bent state.
FIG. 4 illustrates a section view of the second embodiment of the tongue depressor of the present disclosure in the straight state and placed in a human oral cavity.
FIG. 5 illustrates a section view of the second embodiment of the tongue depressor of the present disclosure in the bent state and placed in a human pharynx.
FIG. 6 illustrates a section view of the second embodiment of the tongue depressor of the present disclosure in the bent state after placement of the laryngeal mask.
FIG. 7 illustrates a schematic view and a use state of the third embodiment of the tongue depressor of the present disclosure.
FIG. 8 illustrates a section view of the third embodiment of the tongue depressor of the present disclosure in the use state and placed in a human oral cavity.
FIG. 9 illustrates a section view of the third embodiment of the tongue depressor of the present disclosure in the bent state and placed in a human oral cavity.
FIG. 10 illustrates a section view of the laryngeal mask placed in a human oral cavity alongside the third embodiment of the tongue depressor in the bent state.

### DETAILED DESCRIPTION OF THE EMBODIMENT

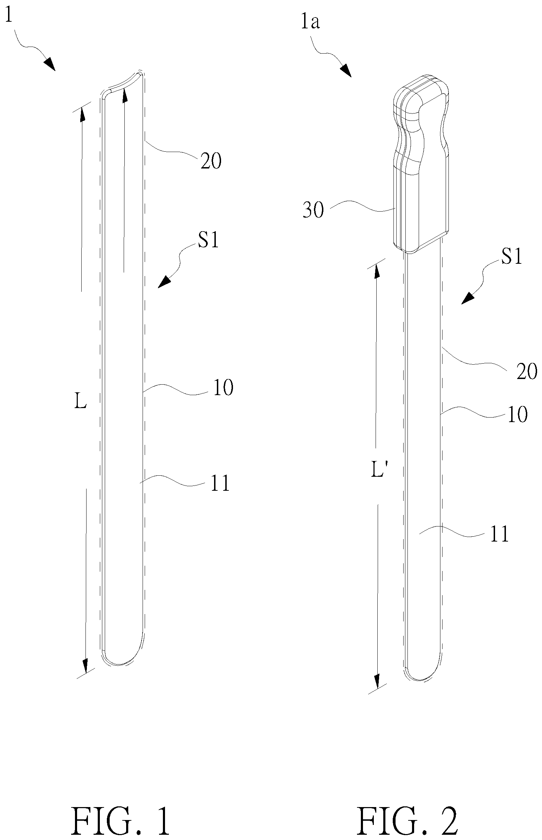

In order to better understand the technical content of the present disclosure, preferred embodiments are described as follows. Please refer to FIG. 1 to FIG. 3, which illustrate schematic views of the first embodiment and the second embodiment of the tongue depressor of the present disclosure in the straight state and a schematic view of the second embodiment of the tongue depressor of the present disclosure in the bent state.

As shown in FIG. 1 to FIG. 3, the tongue depressors 1, 1a of the present disclosure are applied for assisting a placement of a laryngeal mask into a human pharynx. The tongue depressors 1, 1a both include a flexible main body 10, wherein the flexible main body 10 has an elongated and flattened shape with a width (W) and a thickness (D), wherein 1.5 cm < W < 3.5 cm and 0.05 mm < D < 2 mm or 0.05 mm < D < 1.2 mm. The flexible main body 10 includes a convex arc surface 11 and a concave arc surface 12 opposite to the convex arc surface 11. The flexible main body 10 has a straight state S1 and a bent state S2. According to one embodiment of the present disclosure, the flexible main body 10 further has a length (L, L'), where 8 cm < L and L' < 30 cm, and the flexible main body 10 is made of elastic metal (such as spring steel). When an external force is applied to the convex arc surface 11 of the flexible main body 10, the flexible main body 10 transitions from the straight state S1 to the bent state S2. According to one embodiment of the present disclosure, when the flexible main body 10 is subjected to an external force of approximately 0.95 kgw ± 0.5 kgw, the flexible main body 10 transitions from the straight state S1 to the bent state S2.

As shown in FIG. 1 to FIG. 3, the difference between the first embodiment and the second embodiment of the tongue depressor of the present disclosure is that the second embodiment of the tongue depressor 1a has a grip portion 30 for medical personnel to grip. The grip portion 30 is located at one end of the flexible main body 10. It is noted that the width (W) of the flexible main body 10 of the present disclosure refers to the widest portion of the flexible main body 10 itself; therefore, even in the case of the tongue depressor 1a with a handle portion 30, the width (W) of the flexible main body 10 still refers to the widest portion of the flexible main body 10 itself, excluding the width of the handle portion 30. Furthermore, the thickness D of the flexible main body 10 of the present disclosure refers to the thickest portion of the flexible main body 10 itself; therefore, even in the case of the tongue depressor 1a with a handle portion 30, the thickness D of the flexible main body 10 still refers to the thickest portion of the flexible main body 10 itself, excluding the thickness of the handle portion 30. According to a specific embodiment of the present disclosure, the tongue depressor 1 of the present disclosure refers to a slap bracelet commonly found in stationery or toy stores, (slap bracelet, please refer to https://www.youtube.com/watch?v=MF7QlBfmDBI), which remains in a straight line state when no force is applied thereon but bends upon application of force, such as when worn on the wrist or arm. Alternatively, in another embodiment of the present disclosure different from the previous embodiment, one end of the main body 10 of the tongue depressor 1a has a grip portion 30. According to a specific embodiment of the present disclosure, the tongue depressors 1, 1a of the present disclosure further include a cladding 20 for covering the flexible main body 10. The cladding 20 can be made of an elastic material such as silicone, plastic or rubber. It is noted that the thickness (D) of the flexible main body 10 referred to in the present disclosure does not include the thickness of the cladding 20.

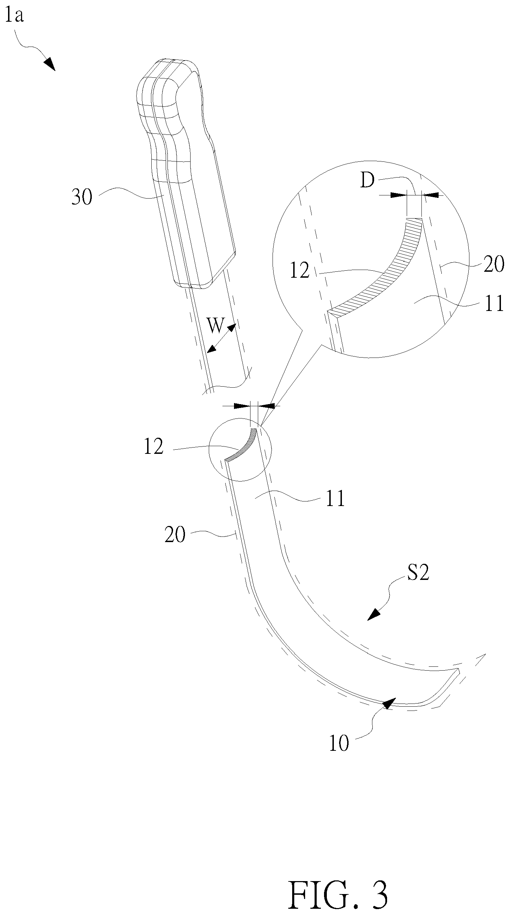

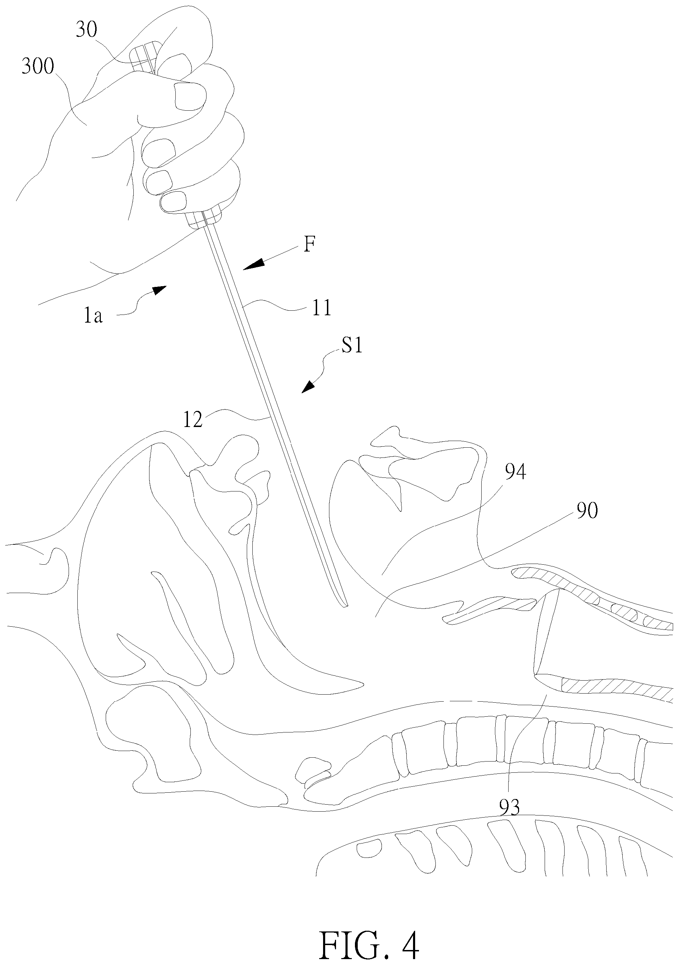

Please refer to FIG. 3 together with FIG. 4 to FIG. 6, which illustrate a section view of the second embodiment of the tongue depressor of the present disclosure in the straight state and placed in a human pharynx, a section view of the second embodiment of the tongue depressor of the present disclosure in the bent state and placed in a human pharynx, and a section view of the second embodiment of the tongue depressor of the present disclosure in the bent state after placement of the laryngeal mask.

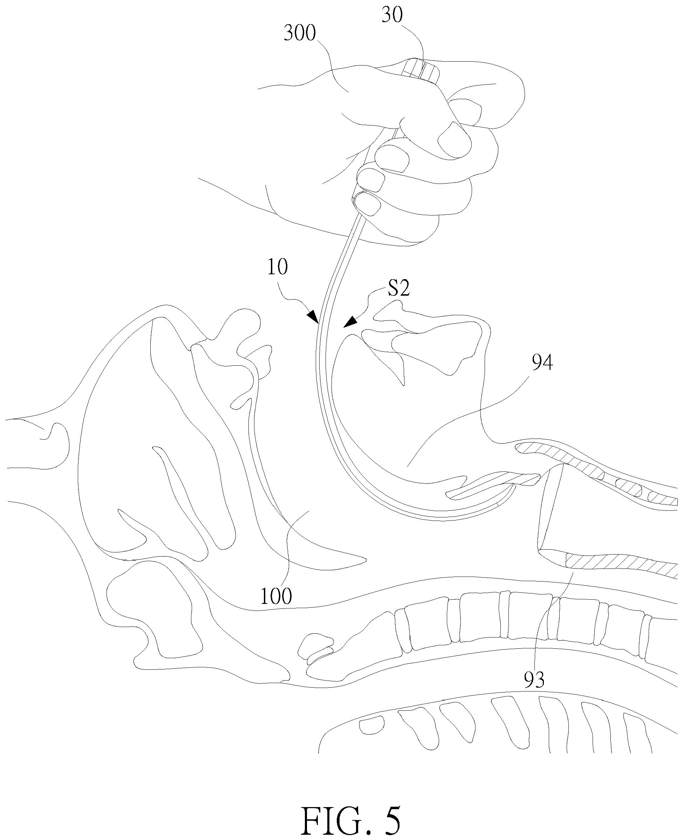

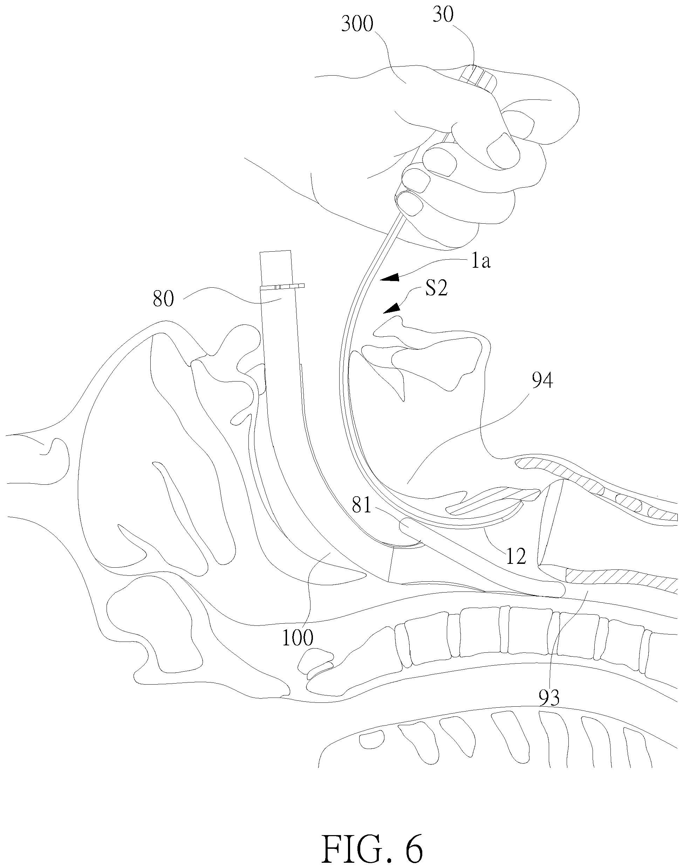

As shown in FIG. 4, when the flexible main body 10 is in the straight state S1, medical personnel 300 can hold the grip portion 30 of the tongue depressor 1a and insert the flexible main body 10 into the human oral cavity 90, and then medical personnel 300 can apply an external force F to the convex arc surface 11 of the flexible main body 10 of the tongue depressor 1a or utilize the external force generated by the contact between the convex arc surface 11 and the teeth, both of which can cause the flexible main body 10 to transition from the straight state S1 to the bent state S2, as shown in FIG. 5. This transition causes the flexible main body 10 in the bent state S2 to depress the tongue base 94 such that a passage 100 is formed between the flexible main body 10 in the bent state S2 and the human oral cavity 90 for allowing the laryngeal mask 80 to enter the human pharynx 93 via the flexible main body 10 and through the passage 100, thus achieving the state shown in FIG. 6. According to a specific embodiment of the present disclosure, the external force required is approximately 0.95 kgw ± 0.5 kgw; however, the present disclosure is not limited to this, as long as the external force is adequate to cause the flexible main body 10 to transition from the straight state S1 to the bent state S2.

More specifically, as shown in FIG. 3 to FIG. 6, the concave arc surface 12 of the flexible main body 10 in the bent state S2 forms the passage 100 with the human hard palate, soft palate, and cervical vertebrae. Due to the slightly higher edges and slightly lower center of the concave arc surface 12, the opening portion 81 of the laryngeal mask 80 contacts the concave arc surface 12 and enters the human pharynx 93 by sliding along the concave arc surface 12 through the passage 100, utilizing the shape characteristics of the concave arc surface 12 to control the direction of movement of the laryngeal mask 80 into the human pharynx 93, thereby reducing the difficulty of placing the laryngeal mask 80 and providing a higher success rate of placement.

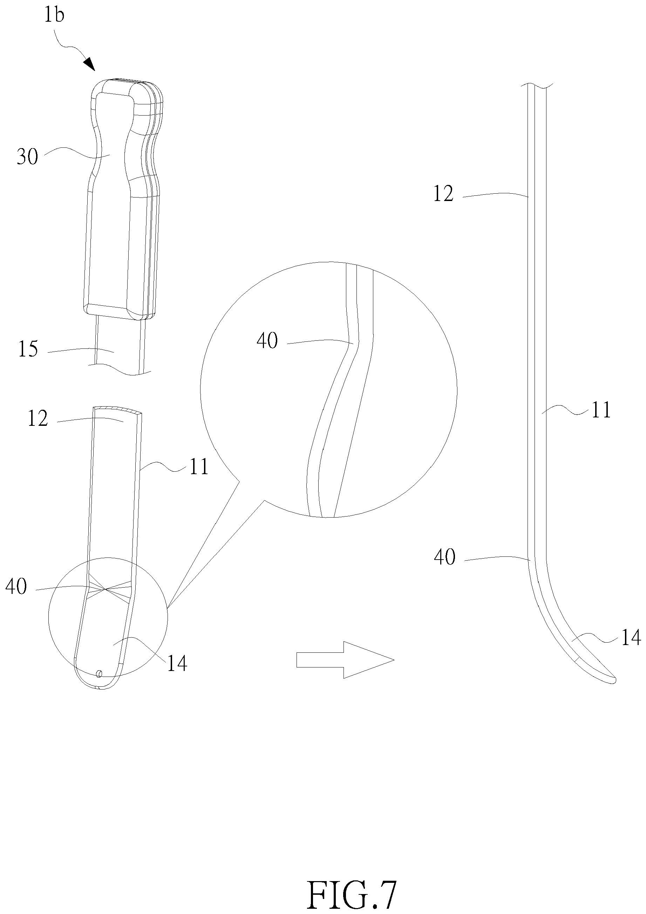

Please refer to FIG. 7 to FIG. 10, which illustrate a schematic view and a use state of the third embodiment of the tongue depressor of the present disclosure, a section view of the third embodiment of the tongue depressor of the present disclosure in the use state and placed in a human pharynx, a section view of the third embodiment of the tongue depressor of the present disclosure in the bent state and placed in a human pharynx, and a section view of the laryngeal mask placed in a human pharynx alongside the third embodiment of the tongue depressor in the bent state.

As shown in FIG. 7, the tongue depressor 1b of the third embodiment of the present disclosure further includes a crease 40 located at a position 1 cm to 5 cm from the front end of the flexible main body 10. The crease 40 is formed by folding the flexible main body 10 from the convex arc surface 11 toward the concave arc surface 12, wherein the crease 40 divides the flexible main body 10 into a first portion 14 and a second portion 15. Before use, bending the first portion 14 toward the convex arc surface 11, and due to the existence and constraint of the crease, the flexible main body cannot be completely bent or curled; i.e., only the first portion 14 can transition to the bent state S2.

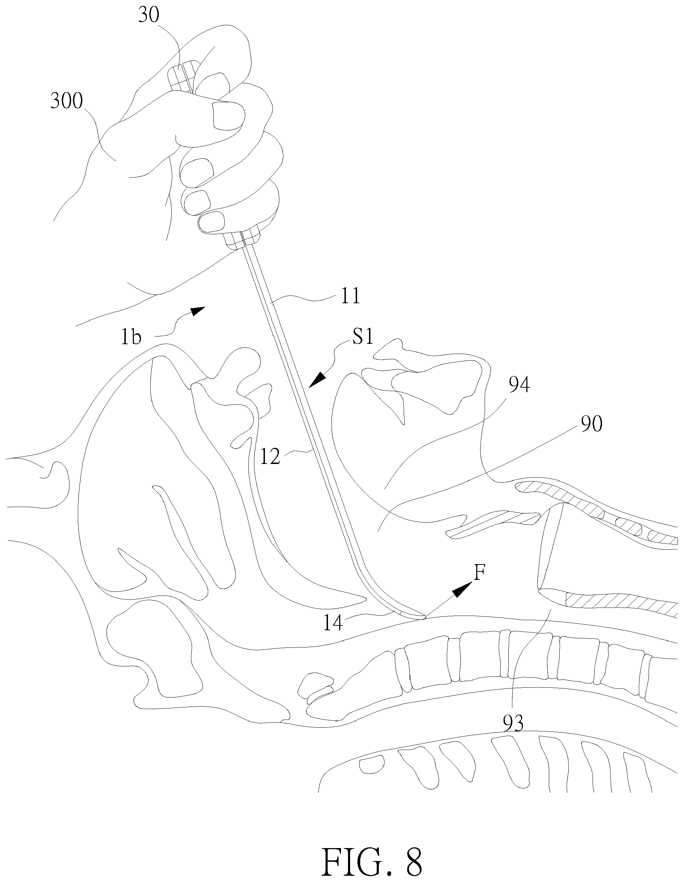

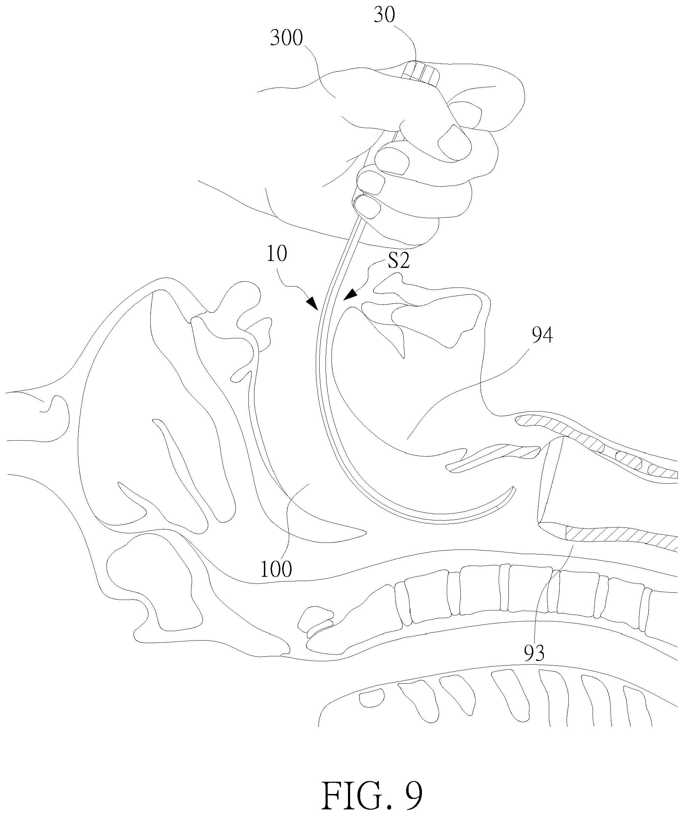

As shown in FIG. 8, in this embodiment, when the flexible main body 10 is in use, medical personnel 300 can hold the grip portion 30 of the tongue depressor 1b and insert the flexible main body 10 into the human oral cavity 90. When the first portion 14 contacts the tissue of the human oral cavity 90, the resilient force F generated by the contact between first portion 14 and the tissue of the human oral cavity 90 is exerted on the convex arc surface 11, and this force causes the second portion 15 of the flexible main body 10 to transition from the straight state S1 to the bent state S2 as shown in FIG. 9. This causes the flexible main body 10 in the bent state S2 to depress the tongue base 94, thereby forming a passage 100 between the flexible main body 10 in the bent state S2 and the human oral cavity 90 such that the laryngeal mask 80 can enter the human pharynx 93 by sliding along the flexible main body 10 and through the passage 100 to achieve the state shown in FIG. 9. In other words, when using the usage state of the flexible main body 10 of the third embodiment of the present disclosure, medical personnel 300 do not need to apply any additional external force to the flexible main body 10. Medical personnel 300 only need to insert the third embodiment of the flexible main body 10 with the first portion 14 in the bent state into the human oral cavity 90 and then utilize the resilient force F exerted when the first portion 14 contacts the tissue of the human oral cavity 90 to cause the flexible main body 10 to transition from the straight state S1 to the bent state S2.

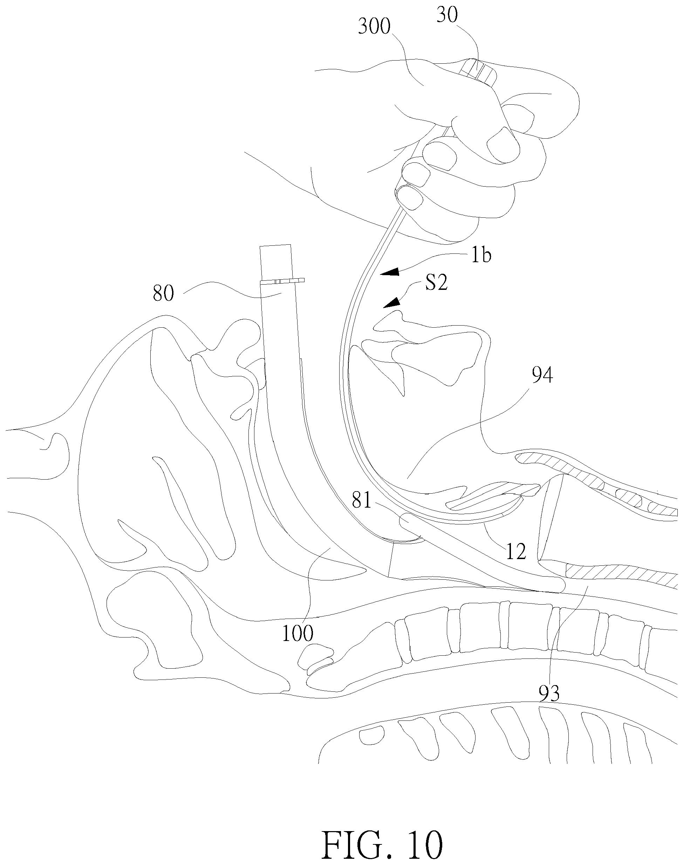

When the flexible main body 10 transitions from the straight state S1 to the bent state S2, as shown in FIG. 10, the concave arc surface 12 of the flexible main body 10 in the bent state S2 forms the passage 100 together with human hard palate, soft palate and cervical vertebrae. Due to the shape of the concave arc surface 12, which is slightly higher on the two opposite sides and slightly lower in the center, the opening portion 81 of the laryngeal mask 80 contacts the concave arc surface 12 and enters the human pharynx 93 by sliding along the concave arc surface 12 through the passage 100, utilizing the shape characteristics of the concave arc surface 12 to control the direction of movement of the laryngeal mask 80 into the human pharynx 93 and thereby reducing the difficulty of placing the laryngeal mask 80 and providing a higher success rate of placement. This resolves the problem mentioned in the prior art where, due to the curved structure of the human upper palate and pharynx, when medical personnel attempt to insert the laryngeal mask through the pharynx into the human body, the front end of the laryngeal mask is often deformed by the pushing force when it comes into contact with the inside of the patient's oral cavity or pharynx, or the soft tissue of the tongue becomes lodged in the opening of the laryngeal mask and renders medical personnel unable to apply force or push the laryngeal mask any farther.

Although the present disclosure has been explained in relation to its preferred embodiments, it is to be understood that many other possible modifications and variations can be made without departing from the spirit and scope of the invention as hereinafter claimed.

## Claims

1. A tongue depressor (1, 1a, 1b), for assisting a placement of a laryngeal mask (80), the tongue depressor (1, 1a, 1b) comprising: a flexible main body (10), being elongated and flattened in shape, with a width (W) and a thickness (D), wherein 1.5 cm < W < 3.5 cm and 0.05 mm < D < 2 mm, the flexible main body (10) having a bent state (S2) and a straight state(S1): whereby, after the flexible main body (10) is placed in a human oral cavity (90) while the tongue depressor (1, 1a, 1b) is in the straight state (S1) and an external force (F) is applied to the flexible main body (10), the flexible main body (10) transitions from the straight state (S1) to the bent state (S2) such that a passage (100) is formed between the flexible main body (10) and the human oral cavity (90) for allowing the laryngeal mask (80) to enter the human pharynx (93) along the flexible main body(10) via the passage(100).

2. The tongue depressor (1, 1a, 1b) as claimed in claim 1, wherein the flexible main body (10) comprises a convex arc surface (11) and a concave arc surface (12) opposite to the convex arc surface (11), wherein when the external force (F) is applied to the convex arc surface (11), the flexible main body (10) transitions from the straight state (S1) to the bent state (S2).

3. The tongue depressor (1, 1a, 1b) as claimed in claim 2, further comprising a crease (40) positioned 1 cm to 5 cm from a front end of the flexible main body (10), wherein the crease (40) is formed by folding the convex arc surface (11) of the flexible main body (10) toward the concave arc surface (12).

4. The tongue depressor (1, 1a, 1b) as claimed in claim 3, wherein the crease (40) divides the flexible main body (10) into a first portion (14) and a second portion (15), wherein the second portion (15) of the flexible main body (10) is in the straight state (S1) and, before the flexible main body (10) is placed into a human oral cavity (90), the first portion (14) is in the bent state (S2).

5. The tongue depressor (1, 1a, 1b) as claimed in claim 4, wherein after the flexible main body (10) has been placed into the human oral cavity (90) and external force (F) is applied to the first portion (14) in the bent state, the second portion (15) of the flexible main body (10) transitions from the straight state (S1) to the bent state (S2) to form a passage (100) between the flexible main body (10) and the human oral cavity (90) for allowing a laryngeal mask (80) to enter the human pharynx (93) by sliding along the flexible main body (10) via the passage (100).

6. The tongue depressor (1, 1a, 1b) as claimed in claim 1, wherein the material of the flexible main body (10) is a metal.

7. The tongue depressor (1, 1a, 1b) as claimed in claim 1, further comprising a cladding (20) covering the flexible main body (10).

8. The tongue depressor (1, 1a, 1b) as claimed in claim 7, wherein the cladding (20) is made of an elastic material.

9. The tongue depressor (1, 1a, 1b) as claimed in any of claims 1 to 8, further comprising a grip portion (30) attached to one end of the flexible main body (10).

10. The tongue depressor (1, 1a, 1b) as claimed in claim 9, wherein the flexible main body (10) has a length (L), where 8 cm < L < 30 cm.
